# EUROPEAN PATENT SPECIFICATION

(11) **EP 4 215 146 B1**
(45) Date of publication and mention of the grant of the patent: **24.09.2025**
(21) Application number: 21868224.3
(22) Date of filing: 05.07.2021
(51) Int. Cl.: A61B 34/30, B25J 9/00, B25J 5/00

(54) **SUSPENSION DISC POSITIONING MECHANISM AND SURGICAL ROBOT**
POSITIONIERMECHANISMUS FÜR EINE AUFHÄNGUNGSSCHEIBE UND CHIRURGISCHER ROBOTER
MÉCANISME DE POSITIONNEMENT DE DISQUE DE SUSPENSION ET ROBOT CHIRURGICAL

(30) Priority: 18.09.2020 CN 202010988531
(43) Date of publication of application: 26.07.2023
(73) Proprietor: Shanghai Microport Medbot (Group) Co., Ltd., Shanghai 201203 (CN)
(72) Inventor: LI, Ming, Shanghai 201203 (CN); YUAN, Shuai, Shanghai 201203 (CN); CHEN, Gong, Shanghai 201203 (CN); HE, Chao, Shanghai 201203 (CN); HE, Yuyuan, Shanghai 201203 (CN)
(74) Representative: Patentship Patentanwaltsgesellschaft mbH
(86) International application number: PCT/CN2021/104608
(87) International publication number: WO 2022/057395

(56) References cited:
- EP-A1- 3 520 729
- WO-A1-2019/128494
- WO-A1-2020/140072
- CN-A- 106 737 850
- CN-A- 108 056 823
- CN-A- 108 186 120
- CN-A- 108 799 351
- CN-A- 110 179 543
- CN-A- 110 179 543
- CN-U- 212 415 897
- US-A1- 2017 191 607
- US-A1- 2017 191 607

## Description

### TECHNICAL FIELD

The present invention relates to the field of medical devices and, in particular to a suspension disc positioning mechanism and a surgical robot.

### BACKGROUND

Most existing minimally invasive surgical robots operate in a master-slave manner, in which under the control of a master console operated by a surgeon, multiple surgical instruments held by respective robot arms of a slave surgical robot are inserted into a patient's body to perform a surgical procedure on a lesion in the patient. As the positions and orientations of the robot arms have a direct impact on smooth performance of the surgical procedure. Before the robot-assisted surgical procedure begins, the surgical robot must be adjusted properly to make it adapted to the procedure to be conducted.

At present, some surgical robot products in the industry are designed for one-by-one adjustment of multiple robot arms mounted on a stationary platform. This design is incapable of quick positioning, and the positioning and working space of robot arms tend to be affected and limited by relative positioning of the operating console and operating cart and are susceptible to the problem of mutual interference.

Some other products employ a single rotatable suspension on which multiple robot arms are mounted and can be collectively adjusted. Although this design allows quick adjustment of the multiple robot arms, it fails to take into account initial orientations of the individual robot arms. Therefore, after the multiple robot arms are coarsely adjusted into a rough range by turning the suspension, it is generally still necessary to finely adjust their orientations arm-by-arm.

Therefore, the existing surgical robots require complicated and time-consuming orientation adjustment of robot arms, which would prolong a surgical procedure.

EP3520729A1 discloses an orienting platform for robotic arms according to the prior art.

### SUMMARY OF THE INVENTION

It is an objective of the present invention to provide a suspension disc positioning mechanism and a surgical robot, which overcomes the problem of complicated and time-consuming orientation adjustment of robot arms as required by the existing surgical robots.

The invention is defined in independent claim 1, further embodiments are described in the dependent claims.

To this end, the present invention provides a suspension disc positioning mechanism including at least two primary suspension discs both attached to a suspension end so as to rotatable about a single primary rotating shaft. Each of the primary suspension discs is configured for attachment of at least one robot arm thereto.

Optionally, the suspension disc positioning mechanism may further include at least one first sub-suspension disc attached to respective at least one of the primary suspension discs so as to be rotatable about a respective sub-rotating shaft parallel to the primary rotating shaft, each of the at least one first sub-suspension disc configured for attachment of at least one robot arm thereto.

Optionally, each of the primary suspension discs may be attached thereto with one first sub-suspension disc and configured for such attachment thereto of one robot arm that the robot arm is rotatable about the sub-rotating shaft.

Optionally, each of the primary suspension discs may extend in a direction perpendicular to the primary rotating shaft, and the suspension disc positioning mechanism may further include at least one second sub-suspension disc each attached to one of the primary suspension discs so as to be movable on this primary suspension disc, each of the at least one second sub-suspension disc configured for attachment of at least one robot arm thereto.

Optionally, each of the primary suspension discs may be attached thereto with at least two second sub-suspension discs, which are spaced apart from each other along an extension direction of the specific primary suspension disc.

Optionally, the suspension disc positioning mechanism may include two primary suspension discs and four second sub-suspension discs, each of the primary suspension discs attached thereto with two of the second sub-suspension discs.

Optionally, each of the at least one second sub-suspension disc may extend in a direction, which is perpendicular to the primary rotating shaft and forms an angle with the direction of extension of the primary suspension disc that the specific second sub-suspension disc is attached to, and may be configured for such attachment thereto of at least one robot arm that each of the at least one robot arm is movable along an extension direction of the specific second sub-suspension disc.

Optionally, each of the primary suspension discs may include a first rail extending along the extension direction of the specific primary suspension disc itself and a first slider disposed so as to be movable on the first rail, and each of the at least one second sub-suspension disc may include a second rail extending along the extension direction of the specific second sub-suspension disc itself and a second slider disposed so as to be movable on the second rail, the first slider attached to a respective one of the at least one second sub-suspension disc, the second slider configured for attachment of a robot arm thereto.

Optionally, when the primary suspension discs are rotating about the primary rotating shaft, an angle between any two of them may be not less than 60°.

Optionally, any two of the primary suspension discs may be provided therebetween with a limiting mechanism for limiting an angle between the two primary suspension discs to a value not less than 60°, the limiting mechanism configured to, when the angle between the primary suspension discs reaches 60° as a result of rotation of a first one of the primary suspension discs relative to a second one of the primary suspension discs, drive the second primary suspension disc to follow the first primary suspension disc to rotate.

Optionally, the suspension disc positioning mechanism may include three primary suspension discs configured to be independently rotatable about the primary rotating shaft, each of the primary suspension discs attached thereto with one first sub-suspension disc configured for attachment thereto with at least one robot arm.

The present invention also provides a surgical robot including the suspension disc positioning mechanism as defined above, a plurality of robot arms and a suspension arm,
One of the primary suspension discs in the suspension disc positioning mechanism is attached to the suspension arm so as to be rotatable about the primary rotating shaft, and each of the other primary suspension disc(s) in the suspension disc positioning mechanism is attached to the primary suspension disc attached to the suspension arm so as to be rotatable about the primary rotating shaft. Moreover, each of the primary suspension discs is attached thereto with at least one robot arm in such a manner that each of the at least one robot arm is rotatable.

In summary, the present invention provides a suspension disc positioning mechanism and a surgical robot. The suspension disc positioning mechanism includes at least two primary suspension discs both attached to a suspension end so as to be rotatable about a single primary rotating shaft. Each of the primary suspension discs is configured for attachment of at least one robot arm thereto.

With this arrangement, the at least two primary suspension discs are attached to the suspension end so as to be both rotatable about the same primary rotating shaft A1 and each allow attachment of at least one robot arm thereto. In this way, by means of the primary suspension discs, multiple robot arms can be quickly adjusted to desired positions in one pass, enabling quick docking of the robot arms. In addition, through providing at least two separate primary suspension discs, each of the robot arms on the primary suspension discs has a larger space for adjustment and an expanded working space.

### BRIEF DESCRIPTION OF THE DRAWINGS

Those of ordinary skill in the art would appreciate that the following drawings are presented merely to enable a better understanding of the present invention rather than to limit the scope thereof in any sense. In the drawings:
Fig. 1 schematically illustrates a surgical scenario of a surgical robot according to an embodiment of the present invention;
Fig. 2 schematically illustrates docking for lateral decubitus positioning according to embodiment of the present invention;
Fig. 3 schematically illustrates docking for supine positioning according to embodiment of the present invention;
Fig. 4 is a schematic illustration of a surgical robot according to a first embodiment of the present invention;
Figs. 5a and 5b are schematic axial cross-sectional views of primary suspension discs and a primary rotating shaft in the first embodiment of the present invention;
Fig. 6 is a schematic illustration of a limiting mechanism in the first embodiment of the present invention;
Fig. 7 is a schematic illustration of a suspension disc positioning mechanism in the first embodiment of the present invention;
Fig. 8 schematically illustrates the suspension disc positioning mechanism in the first embodiment with robot arms attached thereto;
Figs. 9a to 9c schematically illustrate switching of the suspension disc positioning mechanism in the first embodiment of the present invention among different configurations;
Figs. 10a and 10b are schematic illustrations of other preferred examples of the suspension disc positioning mechanism in the first embodiment of the present invention;
Fig. 11 is a schematic illustration of a surgical robot according to a second embodiment of the present invention;
Fig. 12 is a schematic illustration of a suspension disc positioning mechanism in the second embodiment of the present invention;
Fig. 13 schematically illustrates the suspension disc positioning mechanism in the second embodiment with robot arms attached thereto;
Figs. 14a to 14c schematically illustrate switching of the suspension disc positioning mechanism in the second embodiment of the present invention among different configurations;
Fig. 15 is a schematic illustration of a surgical robot according to a third embodiment of the present invention;
Fig. 16 is a schematic illustration of a suspension disc positioning mechanism in thethird embodiment of the present invention with robot arms attached thereto; and
Figs. 17a to 17c schematically illustrate switching of the suspension disc positioning mechanism in the third embodiment of the present invention among different configurations.

In these figures,
1-surgical robot; 2-surgeon console; 3-hospital bed; 4-vision cart; 5-instrument trolley; 6-ventilator and anesthesia machine;
10-suspension disc positioning mechanism; 11-robot arm; 12-suspension arm; 100-primary suspension disc; 110-slewing bearing; 120-first slider; 130-limiting recess; 140-limiting block; 200-first sub-suspension disc; 300-second sub-suspension disc; 320-second slider; A1-primary rotating shaft; A2-sub-rotating shaft.

### DETAILED DESCRIPTION

Objectives, advantages and features of the present invention will become more apparent upon reading the following more detailed description of the present invention, which is set forth by way of particular embodiments with reference to the accompanying drawings. Note that the figures are provided in a very simplified form not necessarily drawn to exact scale and for the only purpose of facilitating easy and clear description of the embodiments. In addition, the structures shown in the figures are usually partial representations of their actual counterparts. In particular, as the figures would have different emphases, they are sometimes drawn to different scales.

As used herein, the singular forms "a", "an" and "the" include plural referents, and the term "or" is generally employed in the sense of "and/or", "several" of "at least one", and "at least two" of "two or more than two". Additionally, the use of the terms "first", "second" and "third" herein is intended for illustration only and is not to be construed as denoting or implying relative importance or as implicitly indicating the numerical number of the referenced item. Accordingly, defining an item with "first", "second" or "third" is an explicit or implicit indication of the presence of one or at least two of the items. The terms "one end" and "the other end", as well as "proximal end" and "distal end", are generally used to refer to opposing end portions including the opposing endpoints, rather than only to the endpoints. The terms "mounting", "coupling" and "connection" should be interpreted in a broad sense. For example, a connection may be a permanent, detachable or integral connection, or a mechanical or electrical connection, or a direct or indirect connection with one or more intervening media, or an internal communication or interaction between two elements. Furthermore, when an element is referred herein to as being disposed on another element, it is typically only meant that the two elements are connected, coupled, mated or geared to each other either directly or indirectly with the presence of intervening element(s). Such reference should not be interpreted as indicating or implying any relative spatial relationship between the two elements. That is, the referenced element may be positioned inside, outside, above, beneath, beside or in any other spatial relationship to the other element, unless the context clearly specifies. Those of ordinary skill in the art can understand the specific meanings of the above-mentioned terms herein, depending on their context.

The present invention principally seeks to provide a suspension disc positioning mechanism and a surgical robot, which overcomes the problem of complicated and time-consuming orientation adjustment of robot arms as required by the existing surgical robots.

A few embodiments will be detailed below with reference to the accompanying drawings.

### EMBODIMENT 1

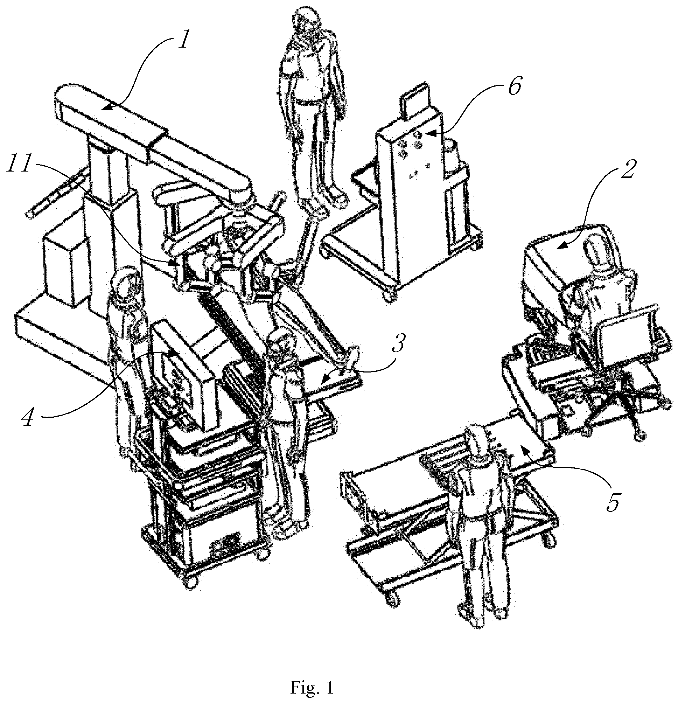

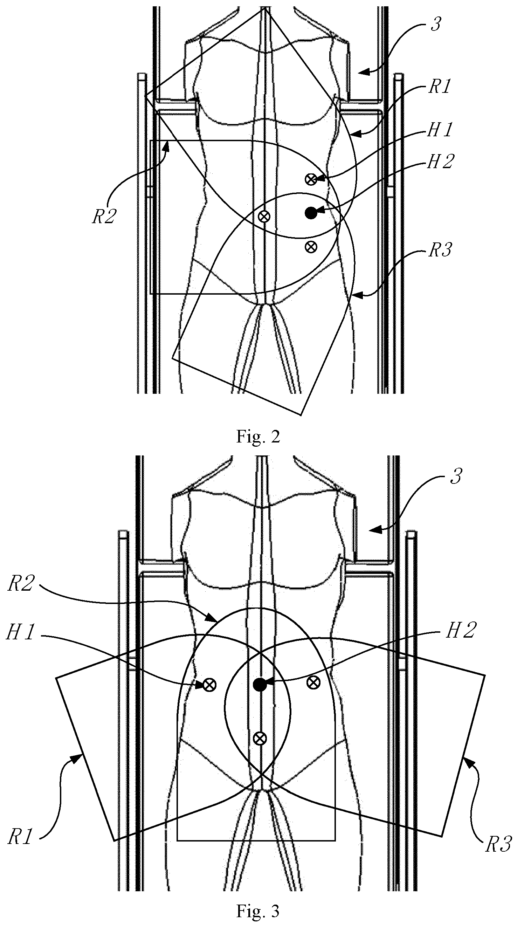

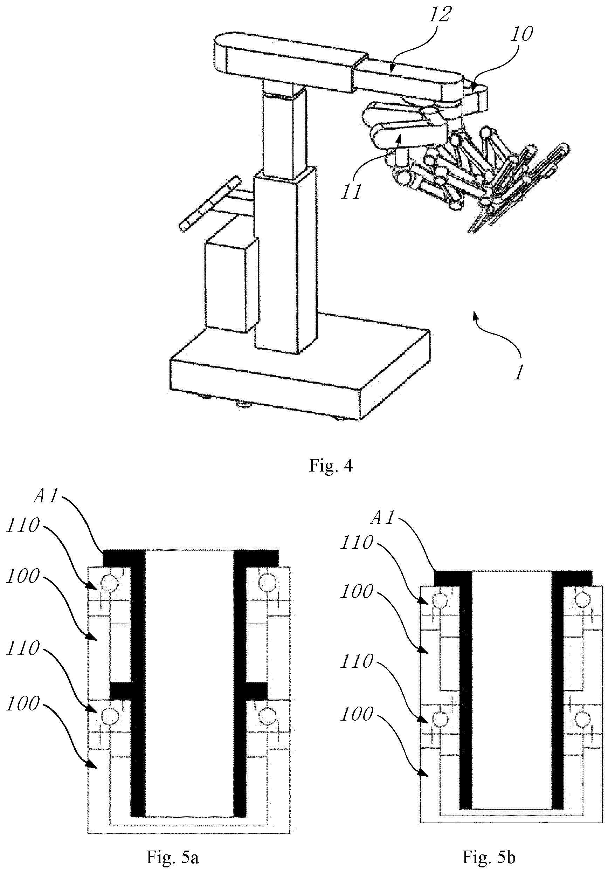

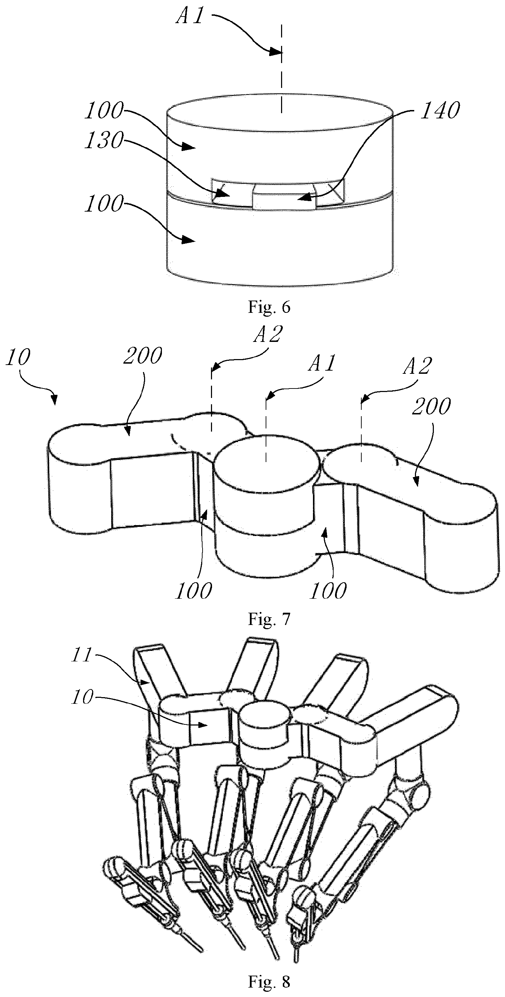

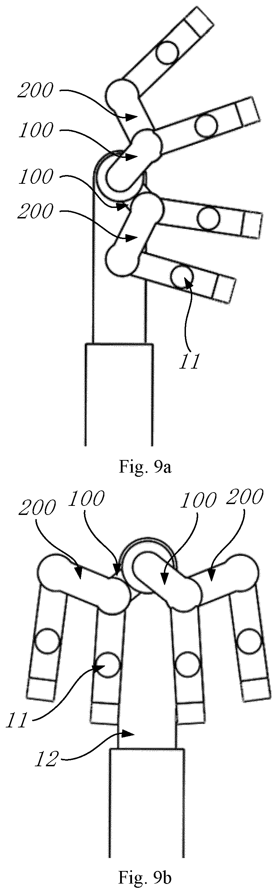

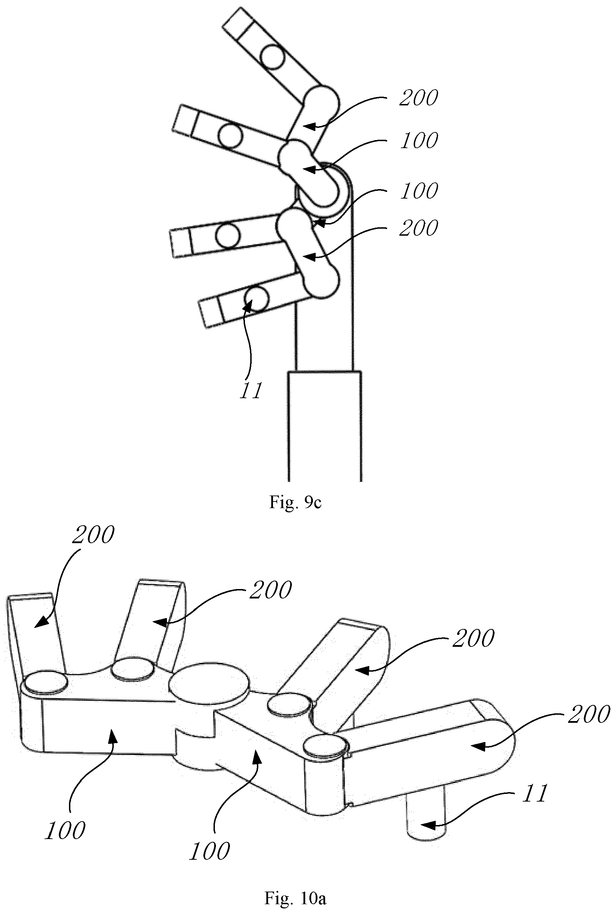

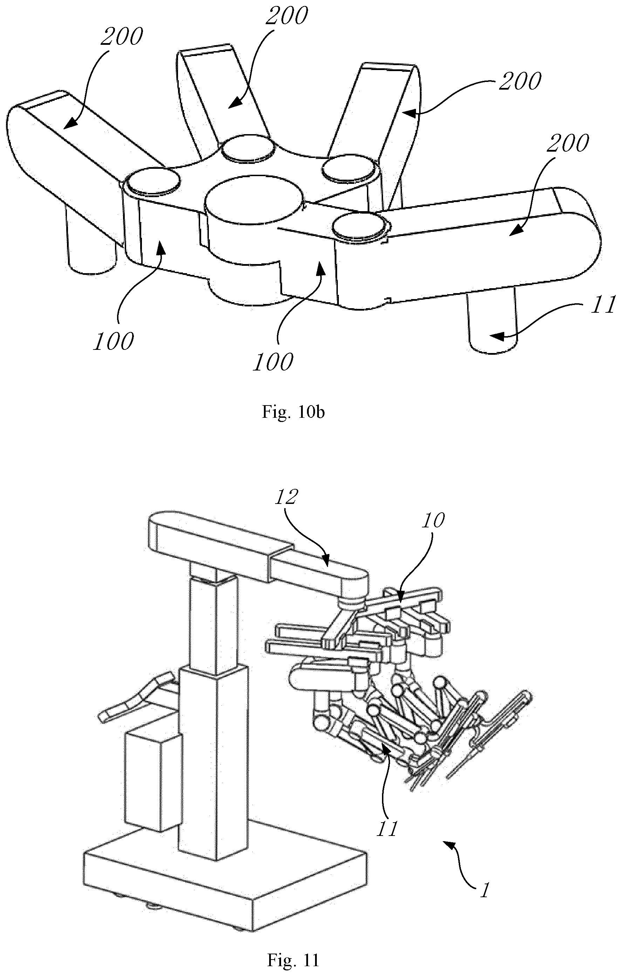

Reference will be made to Figs. 1 to 10b below Fig. 1 schematically illustrates a surgical scenario of a surgical robot according to embodiment of the present invention. Fig. 2 schematically illustrates docking for lateral decubitus positioning according to an embodiment of the present invention. Fig. 3 schematically illustrates docking for supine positioning according to an embodiment of the present invention. Fig. 4 is a schematic illustration of a surgical robot according to a first embodiment of the present invention. Figs. 5a and 5b are schematic axial cross-sectional views of primary suspension discs and a primary rotating shaft in the first embodiment of the present invention. Fig. 6 is a schematic illustration of a limiting mechanism in the first embodiment of the present invention. Fig. 7 is a schematic illustration of a suspension disc positioning mechanism in the first embodiment of the present invention. Fig. 8 schematically illustrates the suspension disc positioning mechanism in the first embodiment of the present invention with robot arms attached thereto. Figs. 9a to 9c schematically illustrate switching of the suspension disc positioning mechanism in the first embodiment of the present invention among different configurations. Figs. 10a and 10b are schematic illustrations of other preferred examples of the suspension disc positioning mechanism in the first embodiment of the present invention.

Embodiments of the present invention provide a surgical robot. Fig. 1 shows an application scenario of the surgical robot in laparoscopic surgery according to an exemplary embodiment. However, the surgical robot of the present invention can also be used in other type of surgery because it is not limited to being used in any particular application. In the following, the surgical robot will be described in the context of use in laparoscopic minimally invasive surgery as an example. However, this shall not be construed as limiting the present invention in any sense.

As shown in Fig. 1, a surgical system includes a surgical robot 1, a surgeon console 2 and a hospital bed 3. Referring to Fig. 4, the surgical robot 1 includes a suspension disc positioning mechanism 10, multiple robot arms 11 and a suspension arm 12. As shown in Fig. 7, the suspension disc positioning mechanism 10 includes at least two primary suspension discs 100, two primary suspension discs 100 both attached to a suspension end rotatable about a single primary rotating shaft A1. Each of the primary suspension discs 100 is configured for attachment of at least one robot arm 11 thereto. The robot arms 11 are equipped with respective different surgical instruments and endoscopes. The surgeon console 2 is provided with master manipulators. Operation of the surgical robot mainly involves effecting minimally invasive surgical treatment on a patient in the hospital bed 3 through remote operation of the surgeon console 2 and the master manipulators by an operator (e.g., a surgeon). The master manipulators control the robot arms 11 and the surgical instruments in such a master-slave manner that, during surgery movement of the robot arms 11 and the surgical instruments follows movement of the master manipulators, which reflects hand movements of the operator. It is to be noted that, in the surgical scenario shown in Fig. 1, the suspension arm 12 of the surgical robot 1 is used as the suspension end. In practice, the suspension end is not limited to the suspension arm 12 of the surgical robot 1. For example, the suspension end can also be a ceiling, a support on the hospital bed 3, or the like. Still alternatively, the suspension disc positioning mechanism 10 may be operably secured to a ceiling, the hospital bed 3 or another support, without departing from the scope of the present invention.

With this arrangement, at least two primary suspension discs 100 are attached to the suspension arm 12 both rotatable about the same primary rotating shaft A1 and each primary suspension disc 100 allow attachment of at least one robot arm 11 thereto. In this way, by means of the primary suspension discs 100, the multiple robot arms 11 can be quickly adjusted to desired positions in one pass, enabling quick docking of the robot arms 11. Through providing at least two separate primary suspension discs 100, each of the robot arms 11 on the primary suspension discs 100 has a larger space for adjustment and an expanded working space.

Optionally, in some embodiments, each primary suspension disc 100 may be directly attached to the primary rotating shaft A1. For example, two primary suspension discs 100 may be provided, as shown in Fig. 5a, and the primary rotating shaft A1 may be a cylinder defining two radially raised rings along its own axis, to which the two respective primary suspension discs 100 are attached by means of respective slewing bearings 110. In some other embodiments, at least one primary suspension disc 100 is directly attached to the primary rotating shaft A1, and the remaining at least one primary suspension disc 100 is hung on the primary suspension disc 100 directly attached to the primary rotating shaft A1. As shown in Fig. 5b, the primary rotating shaft A1 may define one radially raised ring along its own axis, and one primary suspension disc 100 shown as an upper primary suspension disc in Fig. 5b may be attached to the ring by means of its associated slewing bearing 110. Meanwhile, another primary suspension disc 100 shown as a lower primary suspension disc may be attached to the upper primary suspension disc 100 through its associated slewing bearing 110 and thereby hung on the upper primary suspension disc 100.

Preferably, the individual primary suspension discs 100 are rotatable about the primary rotating shaft A1 in such a manner that an angle between any two of the primary suspension discs 100 is kept not less than 60°. In practical use, in order to enable quick docking of the robot arms 11, the two or more primary suspension discs 100 may be configured to be synchronously rotatable about the primary rotating shaft A1 in the same direction. It is to be noted that, here, the ability of the two or more primary suspension disc 100 to synchronously rotate in the same direction means that the primary suspension discs 100 can simultaneously rotate about the primary rotating shaft A1 in the same direction, but does not imply that they must rotate at the same speed. Therefore, in fact, the primary suspension discs 100 may rotate by different angles within the same period of time. Nevertheless, as long as the primary suspension discs 100 are able to synchronously rotate in the same direction, all of the primary suspension discs 100 can quick rotate in the same direction to respective docking positions, thereby enabling simpler and less time-consuming orientation adjustment of the robot arms 11, which can result in time savings for a surgical procedure. However, the synchronous, equidirectional rotation may cause a varying angle between any two of the primary suspension discs 100 when they are rotating at different speeds. Despite this, for more accurate adjustment of the robot arms 11, as long as the angle between them is kept not less than 60° during the rotation, different docking requirements can be satisfied. Subsequently, fine adjustment of any primary suspension disc 100 may be made, for example, by separately rotating it or its robot arm 11, and supplementary orientation adjustment may be made to the robot arms 11. In this way, quick docking can be achieved. Optionally, in some embodiments, the two or more primary suspension disc 100 may be configured to rotate at the same speed. In these cases, any two of the primary suspension discs 100 will be spaced apart by a constant angle when they are both rotating.

Optionally, any two of the primary suspension discs 100 is interposed therebetween with a limiting mechanism for limiting an angle between the two primary suspension discs 100 to a value not less than 60°. The limiting mechanism is configured to, during rotation of a first one of the primary suspension discs 100, upon an angle between the two primary suspension discs 100 reaching 60°, cause a second one of the primary suspension discs 100 to follow the rotation of the first primary suspension disc to rotate. Fig. 6 shows a limiting mechanism interposed between two primary suspension discs 100. The limiting mechanism includes a limiting recess 130 provided in a circumference of a first one of the primary suspension discs 100 and a limiting block 140 attached to a second one of the primary suspension discs 100. The limiting block 140 is movably received in the limiting recess 130. When an angle between the primary suspension discs 100 reaches 60° during rotation, the limiting block 140 will come into abutment against a side wall of the limiting recess 130, thus causing rotation of the second primary suspension discs 100 following the rotation of the first primary suspension disc 100. Generally, the two primary suspension discs 100 can rotate independently about the primary rotating shaft A1 without interfering with each other. However, when one of the primary suspension discs 100 is driven to rotate relative to the other primary suspension disc 100, upon this (active) primary suspension disc 100 reaching a certain angular position (e.g., corresponding to an angle of 60° between the two primary suspension discs 100) and attempting to further approach the other primary suspension disc 100, the limiting mechanism will push the other primary suspension disc 100 so that it follows the active primary suspension disc 100 to rotate synchronously in the same direction. With this arrangement, all the primary suspension discs 100 can be swiftly rotated in the same direction to respective desired docking positions, simplifying orientation adjustment of the robot arms 11, reducing the time required by such adjustment and thereby resulting in time savings for a surgical procedure. Through providing the limiting mechanism(s), it can be ensured that an angle between any two of the primary suspension discs 100 will never become less than 60°. This can minimize interference between the suspension discs or between their robot arms during the adjustment process. It is to be noted that the limiting mechanism(s)is/are not limited as being structured as shown in Fig. 6, and those skilled in the art can select any other suitable mechanical limiting means as actually required.

Optionally, in some surgical procedures, the surgical system further includes a vision cart 4, an instrument trolley 5 and other auxiliary equipment required by the surgical procedures, such as a ventilator and an anesthesia machine 6. Those skilled in the art can select and configure the auxiliary equipment as is conventional, and further description thereof is deemed unnecessary and omitted.

For laparoscopic surgery, there are typically three patterns of patient positioning: left decubitus, right decubitus and supine, which are associated with respective corresponding puncturing patterns. A laparoscopic surgical procedure should take this into account and provide each robot arm 11 with a working space sufficiently covering a target incision site. In preparation for the surgical procedure, the surgical robot is required to swiftly move each robot arm 11 so that it is oriented with an end thereof precisely points toward a target incision site for the specific robot arm 11.

Referring to Fig. 2, in case of left or right decubitus positioning, incisions are made on a corresponding side of a patient's abdomen, and all the suspension discs in the suspension disc positioning mechanism 10 are oriented toward the same side of the patient's body. Moreover, a central column of the surgical robot is positioned beside the hospital bed 3, with surgical instruments corresponding to upper and lower incisions being held by lateral robot arms 11 and an endoscope or surgical instrument corresponding to a middle incision being held by a middle robot arm 11 of the surgical robot.

Referring to Fig. 3, in case of supine positioning, incisions are made in the center of a patient's abdomen vertically in symmetry with respect to his/her sagittal plane. Moreover, the suspension discs of the suspension disc positioning mechanism 10 are arranged along a direction parallel to the patient's sagittal plane, with surgical instruments corresponding to left and right incisions being held by lateral robot arms 11 and an endoscope or surgical instrument corresponding to a middle incision being held by a middle robot arm 11 of the surgical robot.

In order to provide each robot arm 11 with a desirable working space, a range of reach for an endoscope or surgical instrument held by the robot arm 11 should cover, with some margins, a target incision site for the robot arm 11.

Referring to Figs. 7 and 8, a suspension disc positioning mechanism 10 according to a first embodiment includes at least one first sub-suspension disc 200 and two primary suspension discs 100 both attached to a primary rotating shaft A1 so as to be rotatable about a suspension end. Each first sub-suspension disc 200 is configured for attachment of at least one robot arm 11 thereto.

In practical use, the primary suspension discs 100 may be rotated about the primary rotating shaft A1 clockwise or counterclockwise in accordance with an arrangement of the hospital bed 3 to desired angular positions. During the rotation, the first sub-suspension disc(s) 200 attached to the primary suspension discs 100 can follow the primary suspension discs 100 to (passively) rotate over a large angle. Moreover, after the primary suspension discs 100 have been rotated to the desired positions, each first sub-suspension disc 200 may be additionally rotated about an associated sub-rotating shaft A2 in order to affect fine adjustment for tuning a robot arm 11 attached thereto to a more desirable position. It is to be noted that the rotation of the first sub-suspension disc(s) 200 about the respective associated sub-rotating shaft(s) A2 may occur at the same time as the rotation of the primary suspension discs 100 about the primary rotating shaft A1.

Preferably, each of the primary suspension discs 100 is provided with one first sub-suspension disc 200 attached thereto. Each of the primary suspension discs 100 is configured for attachment of one robot arm 11 with rotatability about a sub-rotating shaft A2. In some embodiments, each primary suspension disc 100 may be attached thereto with one robot arm 11. Specifically, the robot arm 11 is rotatably attached to the primary suspension disc 100 around the sub-rotating shaft A2. At the same time, the first sub-suspension disc 200 is also rotatably attached to the primary suspension disc 100 around the sub-rotating shaft A2. Therefore, it can be understood that the first sub-suspension disc 200 and the robot arm 11 attached to the primary suspension disc 100 are respectively attached to the primary suspension disc 100 around the sub-rotating shaft A2. Optionally, the rotation of the first sub-suspension disc 200 is decoupled from and independent of the rotation of the robot arm 11 attachedto the primary suspension disc 100. This can facilitate adjustment of the individual robot arms 11 to their respective desired positions.

With continued reference to Figs. 7 and 8, in one exemplary embodiment, the suspension disc positioning mechanism 10 includes two primary suspension discs 100 and two first sub-suspension discs 200 the respective. Each of the primary suspension discs 100 is attached to one of the first sub-suspension discs 200. Each of the primary suspension discs 100 and the first sub-suspension discs 200 is attached thereto with one robot arm 11. In an exemplary example, each robot arm 11 is able to independently rotate relative to the respective suspension disc (primary suspension disc 100 or sub-suspension disc 200). The robot arms 11 attached to the respective two primary suspension discs 100 are provided mainly for a middle incision and are generally respectively equipped with an endoscope and one surgical instrument. Of course, in some other embodiments, each suspension disc may be alternatively attached thereto with a different number of robot arms 11. Those skilled in the art can configure the number of robot arms 11 attached to each suspension disc as actually required by a surgical procedure.

Switching of the suspension disc positioning mechanism 10 in this embodiment among different configurations will be described in detail below with reference to Figs. 9a to 9c, in conjunction with Fig. 1. Specifically, Fig. 9a shows a configuration of the suspension disc positioning mechanism 10 for left decubitus positioning. Fig. 9b shows a configuration of the suspension disc positioning mechanism 10 for supine positioning. Fig. 9c shows a configuration of the suspension disc positioning mechanism 10 for right decubitus positioning.

As shown in Fig. 1, assuming the central column of the surgical robot 1 is positioned beside a head end of the hospital bed 3, when viewed in the direction from the central column of the surgical robot 1 toward the suspension disc positioning mechanism 10, incisions are generally made on the left side of the suspension arm 12 of the surgical robot 1 in case of left decubitus positioning. Therefore, for left decubitus positioning, the suspension discs are principally deployed on the right side of the suspension arm 12 substantially with the orientations as shown in Fig. 9a, in order not to interfere with or affect a surgical area on the left side of the patient. The robot arms 11 may be docked side by side at positions as required by left decubitus positioning of the patient and oriented toward the incisions.

Figs. 9b and 9c show configurations respectively for supine and right decubitus positioning. For details of these configurations, reference can be made to the foregoing description of the configuration for left decubitus positioning, and further description thereof is omitted here. In practice, the suspension disc positioning mechanism 10 can swiftly switch among these configurations. It is to be noted that, in preparation for a surgical procedure, an initial configuration of the suspension disc positioning mechanism 10 may be any of the configurations of Figs. 9a to 9c, but is not limited to being the configurations of Figs. 9a to 9c. When required by a surgical procedure, the suspension disc positioning mechanism 10 can swiftly switch to a desired configuration for docking.

Referring to Figs. 10a and 10b, in other preferred examples, one or more of the primary suspension discs 100 is each attached thereto with two or more first sub-suspension discs 200.

In the example of Fig. 10a, the suspension disc positioning mechanism 10 includes two primary suspension discs 100 and four first sub-suspension discs 200. Each primary suspension disc 100 is attached thereto with two of the first sub-suspension discs 200. In this case, only the first sub-suspension discs 200 but not the primary suspension discs 100 may be configured for attachment of robot arms 11 thereto. This design of the suspension disc positioning mechanism 10 is further applicable to surgery requiring insertion of surgical instruments into a patient's body from both sides thereof. Specifically, for clinical applications desiring insertion of surgical instruments into the abdominal cavity from both sides thereof, such as hepatobiliary surgery and prostate surgery, after the primary suspension discs 100 have been placed at respective predetermined positions, the first sub-suspension discs 200 on each primary suspension disc 100 may be further rotated and adjusted to position surgical instruments thereon on oppositesides of a patient. This enables the surgical robot 1 to have an expanded scope of applicability

In the example of Fig. 10b, the suspension disc positioning mechanism 10 also includes two primary suspension discs 100 and four first sub-suspension discs 200. However, differing from the example shown in Fig. 8a, three of the first sub-suspension discs 200 are attached to one of the primary suspension discs 100, and the remaining one of the first sub-suspension discs 200 is attached to the other primary suspension disc 100. This design is also further applicable to some types of surgery, additionally expanding the expanded scope of applicability of the surgical robot 1. The primary suspension disc 100 with three first sub-suspension discs 200 attached thereto can collectively drive the three first sub-suspension discs 200 to quickly move to respective desired angular positions, optionally followed by fine adjustment of the individual first sub-suspension discs 200. In this way, much speedier positioning can be achieved.

### EMBODIMENT 2

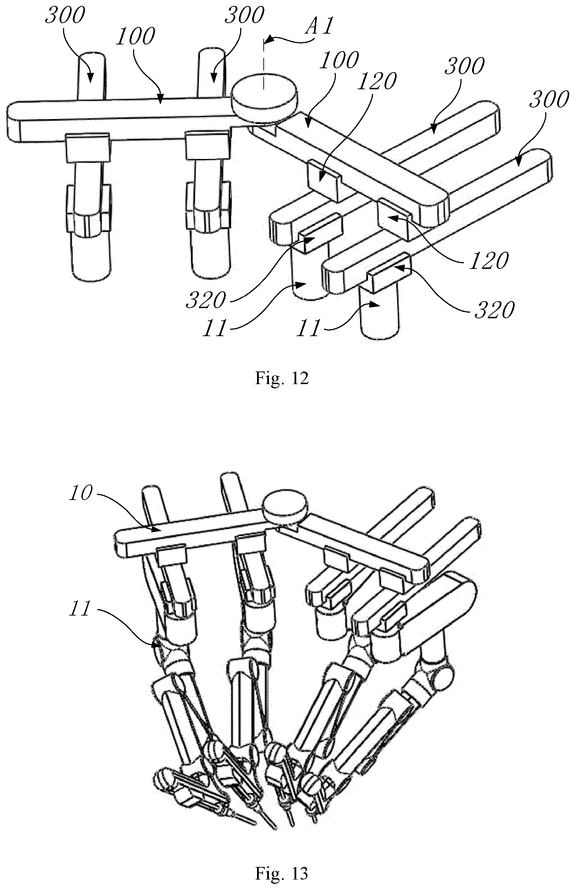

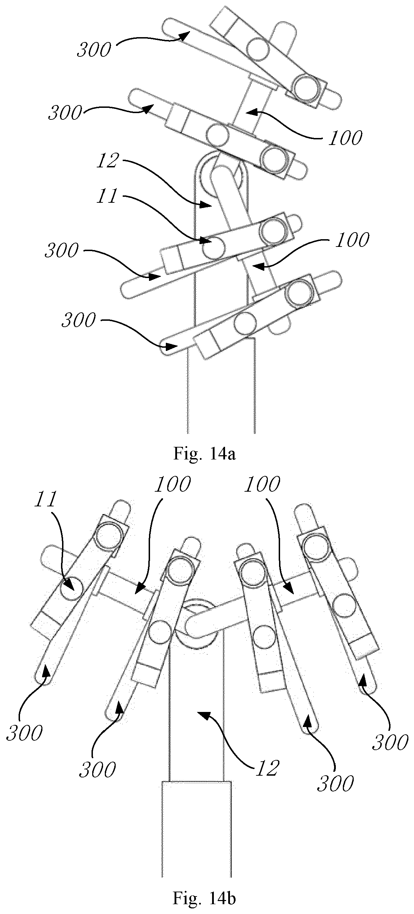

Reference will be made to Figs. 11 to 14c below. Fig. 11 is a schematic illustration of a surgical robot according to a second embodiment of the present invention. Fig. 12 is a schematic illustration of a suspension disc positioning mechanism in the second embodiment of the present invention. Fig. 13 schematically illustrates the suspension disc positioning mechanism in the second embodiment of the present invention with robot arms attached thereto. Figs. 14a to 14c schematically illustrate switching of the suspension disc positioning mechanism in the second embodiment of the present invention among different configurations.

The suspension disc positioning mechanism and surgical robot of the second embodiment are substantially similar to those of the first embodiment. In the following, only features unique to the second embodiment will be described, and those common to the first and second embodiments that have been described above will not be described again.

As shown in Figs. 11 to 13, the suspension disc positioning mechanism 10 in the second embodiment includes primary suspension discs 100 each extending in a direction perpendicular to a primary rotating shaft A1. The suspension disc positioning mechanism 10 further includes at least one second sub-suspension disc 300 each attached to a respective one of the primary suspension discs 100 so as to be movable in the direction of extension of the primary suspension disc 100. Each second sub-suspension disc 300 is configured for attachment of at least one robot arm 11 thereto. It is to be noted that each primary suspension disc 100 is not limited to extending along a straight line as shown in Figs. 9 to 11; rather, it may extend along a curve line, such as an arc, which is perpendicular to the primary rotating shaft A1. With this arrangement, the second sub-suspension disc 300 may be movable along the curved contour of the primary suspension disc 100.

In practical use, the primary suspension discs 100 may be rotated about the primary rotating shaft A1 clockwise or counterclockwise in accordance with an arrangement of the hospital bed 3 to desired angular positions. During the rotation, the second sub-suspension disc(s) 300 attached to the primary suspension discs 100 can follow the primary suspension discs 100 to (passively) rotate over a large angle. Moreover, after the primary suspension discs 100 have been rotated to the desired positions, each second sub-suspension disc 300 may be additionally moved on the corresponding primary suspension disc 100 in order to affect fine adjustment for tuning the robot arm(s) 11 attached thereto to more desirable position(s). It is to be noted that the movement of each second sub-suspension disc 300 on the corresponding primary suspension disc 100 may occur at the same time as the rotation of the primary suspension discs 100 about the primary rotating shaft A1.

In some embodiments, each robot arm 11 is rotatably attached to one of the second sub-suspension disc(s) 300 and move with the corresponding second sub-suspension disc(s) 300 on the corresponding primary suspension disc 100. In some other embodiments, each robot arm 11 may also be movable on the second sub-suspension disc 300, to which it is attached. Preferably, each second sub-suspension disc 300 extends in a direction, which is perpendicular to the primary rotating shaft A1. Eachsecond sub-suspension disc 300 forms an angle with the direction of extension of the corresponding primary suspension disc 100. Each second sub-suspension disc 300 is configured for such attachment of at least one robot arm 11 that each robot arm 11 is movable on the second sub-suspension disc 300. Thus, the direction of extension of each second sub-suspension disc 300 forms an angle with the direction of extension of the corresponding primary suspension disc 100, with each robot arm 11 attached to the second sub-suspension disc 300 being able to move thereon and with the second sub-suspension disc 300 itself being able to move on the corresponding primary suspension disc 100. With this arrangement, each robot arm 11 can have more degrees of freedom of adjustment. Likewise, each second sub-suspension disc 300 is not limited to having any particular shape, and it may have a linear shape, as shown in Figs. 11 to 13, or any other suitable shape, such as an arcuate shape.

Optionally, each primary suspension disc 100 may include a first rail extending along a length of the primary suspension disc 100 and first slider 120 movable on the first rail. Moreover, each second sub-suspension disc 300 may include a second rail extending along a length of the second sub-suspension disc 300 and a second slider 320 movable on the second rail. Each second sub-suspension disc 300 may be attached to the first slider 120 of the corresponding primary suspension disc 100, and the second slider 320 may be configured for attachment of a robot arm 11 thereto. In one exemplary embodiment, each second sub-suspension disc 300 may be attached to the corresponding primary suspension disc 100 by the first slider 120 and the first rail thereof, and each robot arm 11 may be attached to one of the second sub-suspension disc(s) 300 by the second slider 320 and the second rail thereof. In this way each robot arm 11 can be adjusted relative to the corresponding primary suspension disc 100 in both the directions of the corresponding first and second rails. Further, each robot arm 11 may be rotatably coupled to the corresponding second slider 320 and thus have even more degrees of freedom of adjustment. Of course, in some other embodiments, the robot arm(s) 11, the second sub-suspension disc(s) 300 and the primary suspension discs 100 are not limited to being interconnected by the sliders and rails, and timing belts, lead screws and other coupling means for power transmission well known in the art are also possible within the scope of the present invention.

Preferably, each of the primary suspension discs 100 is attached thereto with at least two second sub-suspension discs 300, which are spaced apart from each other along the extension direction of the primary suspension disc 100. In an exemplary example, the suspension disc positioning mechanism 10 includes two primary suspension discs 100 and four second sub-suspension discs 300, each of the primary suspension discs 100 is attached thereto with two of the second sub-suspension discs 300. Each primary suspension disc 100 is configured for indirect attachment of robot arms 11 thereto through the second sub-suspension discs 300. That is, the robot arms 11 are not directly provided on the primary suspension disc 100. Each second sub-suspension disc 300 is attached thereto with one robot arm 11. In use, robot arms 11 attached to the two second sub-suspension discs 300 proximal to the primary rotating shaft A1 are intended mainly for a middle incision and generally mounted respectively with an endoscope and one surgical instrument, while robot arms 11 attached to the two second sub-suspension discs 300 distal from the primary rotating shaft A1 are intended mainly for lateral incisions and generally mounted with respective surgical instruments. Of course, in some other embodiments, each second sub-suspension disc 300 may be attached thereto with a different number of robot arms 11, and some robot arms 11 may also be provided on the primary suspension discs 100. Those skilled in the art may configure the number and locations of robot arms 11 provided on each suspension disc as required by a surgical procedure, and the present invention is not limited in this regard.

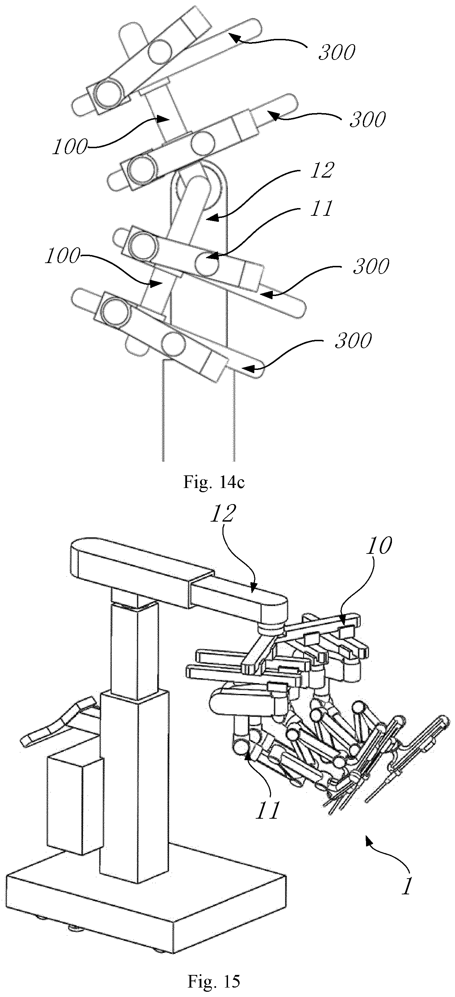

Reference is now made to Figs. 14a to 14c. Fig. 14a shows a configuration of the suspension disc positioning mechanism 10 in the second embodiment for left decubitus positioning. Fig. 14b shows a configuration of the suspension disc positioning mechanism 10 in the second embodiment for supine positioning. Fig. 14c shows a configuration of the suspension disc positioning mechanism 10 in the second embodiment for right decubitus positioning. For details of these configurations, reference can be made to the foregoing description of the configurations in the first embodiment, and further description thereof is omitted here. In practice, the suspension disc positioning mechanism 10 can swiftly switch among these configurations. It is to be noted that, in preparation for a surgical procedure, an initial configuration of the suspension disc positioning mechanism 10 may be, but is not limited to being, any of the configurations of Figs. 14a to 14c. When required by a surgical procedure, the suspension disc positioning mechanism 10 can swiftly switch to a desired configuration for docking.

### EMBODIMENT 3

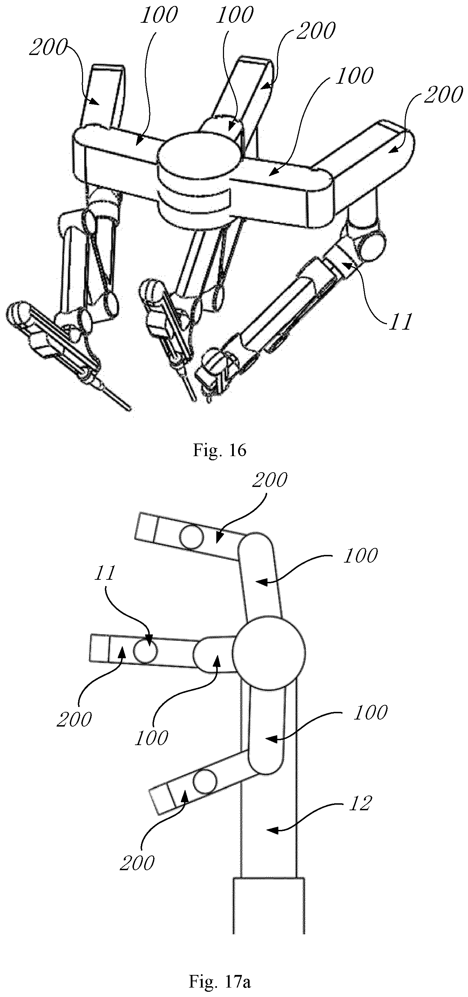

Reference will be made to Figs. 15 to 17c below Fig. 15 is a schematic illustration of a surgical robot according to a third embodiment of the present invention. Fig. 16 is a schematic illustration of a suspension disc positioning mechanism in the third embodiment of the present invention with robot arms attached thereto. Figs. 17a to 17c schematically illustrate switching of the suspension disc positioning mechanism in the third embodiment of the present invention among different configurations.

The suspension disc positioning mechanism and surgical robot of the third embodiment are substantially similar to those of the first embodiment. In the following, only features unique to the third embodiment will be described, and those common to the first and third embodiments that have been described above will not be described again.

As shown in Figs. 15 to 17c, the suspension disc positioning mechanism 10 in the third embodiment includes three primary suspension discs 100 includes three primary suspension discs 100 each configured to be independently rotatably about a primary rotating shaft A1.

Preferably, the suspension disc positioning mechanism 10 further include three first sub-suspension discs 200, which are attached to the respective three primary suspension discs 100 so as to be each rotatable about a respective sub-rotating shaft A2. Each of the primary suspension discs 100 is configured to indirect attachment of a robot arm 11 thereto through the respective first sub-suspension disc 200. That is, the robot arm 11 is not directly provided on the primary suspension disc 100. Each first sub-suspension disc 200 is attached thereto with one robot arm 11. The robot arm 11 is preferably rotatably attached to the first sub-suspension disc 200.

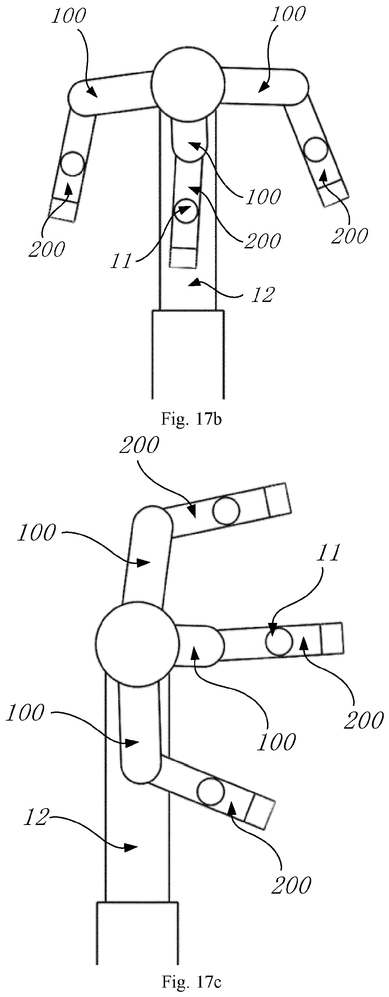

Reference is now made to Figs. 17a to 17c. Fig. 17a shows a configuration of the suspension disc positioning mechanism 10 in the third embodiment for left decubitus positioning. Fig. 17b shows a configuration of the suspension disc positioning mechanism 10 in the third embodiment for supine positioning. Fig. 17c shows a configuration of the suspension disc positioning mechanism 10 in the third embodiment for right decubitus positioning. For details of these configurations, reference can be made to the foregoing description of the configurations in the first embodiment, and further description thereof is omitted here. In practice, the suspension disc positioning mechanism 10 can swiftly switch among these configurations. It is to be noted that, in preparation for a surgical procedure, an initial configuration of the suspension disc positioning mechanism 10 may be any of the configurations of Figs. 17a to 17c, but is not limited to the configurations of Figs. 17a to 17c. When required by a surgical procedure, the suspension disc positioning mechanism 10 can swiftly switch to a desired configuration for docking.

Optionally, at least some of the primary suspension discs 100 or first sub-suspension discs 200 may be provided with clutch mechanisms, which allow the shaft of each suspension disc to be linked to or released from another suspension disc. In some cases, when it is necessary to make a positional or angular adjustment to a suspension disc, a corresponding clutch mechanism may temporarily unlink the suspension disc from another suspension disc to allow the positional or angular adjustment to be made. For example, in general cases, in order to enable a swift configuration switch, the three primary suspension discs 100 may be interlinked by clutch mechanisms. Once they are rotated to a desired location, the clutch mechanisms may be manipulated to release one or two of the primary suspension discs 100 to enable its or they independent movability, which can facilitate fine adjustment.

It is to be noted that the features of the above embodiments may be used in combination. For example, the suspension disc positioning mechanism 10 may include two primary suspension discs 100, one of which is configured for rotatable attachment of a sub-suspension disc 200 thereto as in the first embodiment, and the other is configured for movable attachment of a sub-suspension disc 200 thereto as in the second embodiment. In use, after the two primary suspension discs 100 are rotated to respectivedesired positions, the sub-suspension discs attached respective thereto may be finely adjusted in different manners. For more details in this regard, reference can be made to the foregoing description, and further description thereof is omitted.

In summary, the present invention provides a suspension disc positioning mechanism and a surgical robot. The suspension disc positioning mechanism includes at least two primary suspension discs both attached to a suspension end so as to be rotatable about a single primary rotating shaft. Each of the primary suspension discs is configured for attachment of at least one robot arm thereto. With this arrangement, the at least two primary suspension discs are attached to the suspension end so as to be both rotatable about the same primary rotating shaft A1 and each allow attachment of at least one robot arm thereto. In this way by means of the primary suspension discs, multiple robot arms can be quickly adjusted to desired positions in one pass, enabling quick docking of the robot arms. In addition, through providing at least two separate primary suspension discs, each of the robot arms on the primary suspension discs has a larger space for adjustment and an expanded working space.

The description presented above is merely that of a few preferred embodiments of the present invention and is not intended to limit the scope thereof in any sense. Any and all changes and modifications made by those of ordinary skill in the art based on the above teachings fall within the scope as defined in the appended claims.

## Claims

1. A suspension disc positioning mechanism (10), comprising at least two primary suspension discs (100) with lateral arms, two of the primary suspension discs (100) both rotatably attached to a suspension end around a single primary rotating shaft (A1), ends of the arms of the primary suspension discs (100) configured for attachment of at least one robot arm (11) thereto, **characterized in that**:
any two of the primary suspension discs (100) are provided therebetween with a limiting mechanism, the limiting mechanism configured to, when an angle between the primary suspension discs (100) reaches a certain value as a result of rotation of a first one of the primary suspension discs (100) relative to a second one of the primary suspension discs (100), drive the second primary suspension disc (100) to follow the first primary suspension disc (100) to rotate.

2. The suspension disc positioning mechanism (10) according to claim 1, further comprising at least one first sub-suspension disc (200), the first sub-suspension disc(s) (200) rotatably attached to respective primary suspension disc(s) (100) around a respective sub-rotating shaft (A2), the sub-rotating shaft parallel to the primary rotating shaft (A1), at least one of the primary suspension discs (100) attached to the at least one first sub-suspension disc (200), each of the at least one first sub-suspension disc (200) respectively configured for attachment of the at least one robot arm (11) thereto.

3. The suspension disc positioning mechanism (10) according to claim 2, wherein each of said primary suspension discs (100) is attached thereto with one of the first sub-suspension disc(s) (200), and each first sub-suspension disc (200) configured for rotatably attachment thereto of one robot arm (11) around the sub-rotating shaft (A2).

4. The suspension disc positioning mechanism (10) according to claim 1, wherein each of the primary suspension discs (100) extends in a direction perpendicular to the primary rotating shaft (A1), the suspension disc positioning mechanism (10) further comprising at least one second sub-suspension disc (300) each movably attached to one of the primary suspension discs (100) along an extension direction of the primary suspension disc (100), each of said second sub-suspension disc (300) configured for attachment of at least one robot arm (11) thereto.

5. The suspension disc positioning mechanism (10) according to claim 4, wherein each of the primary suspension discs (100) is attached thereto with at least two second sub-suspension discs (300), the second sub-suspension discs (300) attached to the same primary suspension disc (100) are spaced apart from each other along the extension direction of the specific primary suspension disc (100).

6. The suspension disc positioning mechanism (10) according to claim 5, comprising two primary suspension discs (100) and four second sub-suspension discs (300), each of the primary suspension discs (100) attached thereto with two of the second sub-suspension discs (300).

7. The suspension disc positioning mechanism (10) according to claim 4, wherein said second sub-suspension discs (300) extend in a direction perpendicular to the primary rotating shaft (A1), an extension direction of the second sub-suspension disc (300) arranged at an angle with the extension direction of the specific primary suspension disc (100), and each second sub-suspension disc (300) configured for movably attachment thereto of at least one robot arm (11) along the extension direction of the specific second sub-suspension disc (300).

8. The suspension disc positioning mechanism (10) according to claim 7, wherein said primary suspension discs (100) comprise a first rail extending along an extension direction of the specific primary suspension disc (100) itself and a first slider (120) disposed so as to be movable on the first rail; wherein said second sub-suspension disc (300) comprise a second rail extending along an extension direction of the specific second sub-suspension disc (300) itself and a second slider (320) disposed so as to be movable on the second rail, said second sub-suspension discs (300) attached to the first slider (120), the second slider (320) configured for attachment of a robot arm (11) thereto.

9. The suspension disc positioning mechanism (10) according to claim 1, wherein when the primary suspension discs (100) are rotating around the primary rotating shaft (A1), the angle between any two of the primary suspension discs (100) is not less than 60°.

10. The suspension disc positioning mechanism (10) according to claim 1, wherein the limiting mechanism is configured to, when the angle between the primary suspension discs (100) reaches 60° as the result of rotation of the first one of the primary suspension discs (100) relative to the second one of the primary suspension discs (100), drive the second primary suspension disc (100) to follow the first primary suspension disc (100) to rotate.

11. The suspension disc positioning mechanism (10) according to claim 1, comprising three primary suspension discs (100) configured to be independently rotatable around the primary rotating shaft (A1), each of the primary suspension discs (100) attached thereto with one first sub-suspension disc (200), each first sub-suspension disc configured for attachment thereto with at least one robot arm (11).

12. The suspension disc positioning mechanism (10) according to claim 1, wherein at least one of the primary suspension discs (100) is provided with a clutch mechanism, which allows the primary rotating shaft (A1) of each primary suspension disc (100) to be linked to or released from another primary suspension disc (100).

13. The suspension disc positioning mechanism (10) according to claim 2, comprising at least two first sub-suspension disc (200), wherein the first sub-suspension disc(s) (200) is(are) provided with a clutch mechanism, which allows the sub-rotating shaft (A2) of each first sub-suspension disc (200) to be linked to or released from another first sub-suspension disc (200).

14. The suspension disc positioning mechanism (10) according to claim 1, wherein the at least two primary suspension discs (100) are configured to be synchronously rotatable around the primary rotating shaft (A1) in the same direction.

15. A surgical robot (1), comprising the suspension disc positioning mechanism (10) according to any one of claims 1 to 14, a plurality of robot arms (11) and a suspension arm (12),
wherein one of the primary suspension discs (100) with lateral arms in the suspension disc positioning mechanism (10) is rotatably attached to the suspension arm (12) around the primary rotating shaft (A1), wherein each of the other primary suspension disc(s) (100) with lateral arms in the suspension disc positioning mechanism (10) is rotatably attached to the primary suspension disc (100) attached to the suspension arm (12) around the primary rotating shaft (A1), and wherein ends of the arms of the primary suspension discs (100) are attached thereto with at least one robot arm (11), and each of said robot arms (11) is rotatably attached to the corresponding primary suspension disc (100).

## Patentansprüche

1. Aufhängungsscheiben-Positionierungsmechanismus (10), umfassend mindestens zwei primäre Aufhängungsscheiben (100) mit seitlichen Armen, wobei beide von zwei der primären Aufhängungsscheiben (100) drehbar an einem Aufhängungsende um eine einzelne primäre Drehwelle (A1) befestigt sind, wobei Enden der Arme der primären Aufhängungsscheiben (100) zur Befestigung mindestens eines Roboterarms (11) daran ausgebildet sind, **dadurch gekennzeichnet, dass**:
zwischen zwei beliebigen der primären Aufhängungsscheiben (100) ein Begrenzungsmechanismus vorgesehen ist, wobei der Begrenzungsmechanismus ausgebildet ist, wenn ein Winkel zwischen den primären Aufhängungsscheiben (100) infolge der Drehung einer ersten der primären Aufhängungsscheiben (100) relativ zu einer zweiten der primären Aufhängungsscheiben (100) einen bestimmten Wert erreicht, die zweite primäre Aufhängungsscheibe (100) anzutreiben, der ersten primären Aufhängungsscheibe (100) zu folgen, sich zu drehen.

2. Aufhängungsscheiben-Positionierungsmechanismus (10) nach Anspruch 1, ferner umfassend mindestens eine erste Unteraufhängungsscheibe (200), wobei die erste(n) Unteraufhängungsscheibe(n) (200) drehbar an der/den jeweiligen primären Aufhängungsscheibe(n) (100) um eine jeweilige Unterdrehwelle (A2) befestigt ist/sind, wobei die Unterdrehwelle parallel zu der primären Drehwelle (A1) ist, wobei mindestens eine der primären Aufhängungsscheiben (100) an der mindestens einen ersten Unteraufhängungsscheibe (200) befestigt ist, wobei jede der mindestens einen ersten Unteraufhängungsscheiben (200) jeweils zur Befestigung des mindestens einen Roboterarms (11) daran ausgebildet ist.

3. Aufhängungsscheiben-Positionierungsmechanismus (10) nach Anspruch 2, wobei an jeder der primären Aufhängungsscheiben (100) eine der ersten Unteraufhängungsscheiben (200) befestigt ist, und wobei jede erste Unteraufhängungsscheibe (200) zur drehbaren Befestigung eines Roboterarms (11) daran um die Unterdrehwelle (A2) ausgebildet ist.

4. Aufhängungsscheiben-Positionierungsmechanismus (10) nach Anspruch 1, wobei sich jede der primären Aufhängungsscheiben (100) in einer Richtung senkrecht zur primären Drehwelle (A1) erstreckt, wobei der Aufhängungsscheiben-Positionierungsmechanismus (10) ferner mindestens eine zweite Unteraufhängungsscheibe (300) umfasst, die jeweils beweglich an einer der primären Aufhängungsscheiben (100) entlang einer Erstreckungsrichtung der primären Aufhängungsscheibe (100) befestigt ist, wobei jede der zweiten Unteraufhängungsscheiben (300) zur Befestigung mindestens eines Roboterarms (11) daran ausgebildet ist.

5. Aufhängungsscheiben-Positionierungsmechanismus (10) nach Anspruch 4, wobei an jeder der primären Aufhängungsscheiben (100) mindestens zwei zweite Unteraufhängungsscheiben (300) befestigt sind, wobei die an derselben primären Aufhängungsscheibe (100) befestigten zweiten Unteraufhängungsscheiben (300) entlang der Erstreckungsrichtung der jeweiligen primären Aufhängungsscheibe (100) voneinander beabstandet sind.

6. Aufhängungsscheiben-Positionierungsmechanismus (10) nach Anspruch 5, umfassend zwei primäre Aufhängungsscheiben (100) und vier zweite Unteraufhängungsscheiben (300), wobei an jeder der primären Aufhängungsscheiben (100) zwei der zweiten Unteraufhängungsscheiben (300) befestigt sind.

7. Aufhängungsscheiben-Positionierungsmechanismus (10) nach Anspruch 4, wobei sich die zweiten Unteraufhängungsscheiben (300) in einer Richtung senkrecht zur primären Drehwelle (A1) erstrecken, wobei eine Erstreckungsrichtung der zweiten Unteraufhängungsscheibe (300) in einem Winkel zur Erstreckungsrichtung der spezifischen primären Aufhängungsscheibe (100) angeordnet ist, und wobei jede zweite Unteraufhängungsscheibe (300) zur Befestigung mindestens eines Roboterarms (11) daran entlang der Erstreckungsrichtung der spezifischen zweiten Unteraufhängungsscheibe (300) ausgebildet ist.

8. Aufhängungsscheiben-Positionierungsmechanismus (10) nach Anspruch 7, wobei die primären Aufhängungsscheiben (100) eine erste Schiene umfassen, die sich entlang einer Erstreckungsrichtung der spezifischen primären Aufhängungsscheibe (100) selbst erstreckt, sowie einen ersten Gleiter (120), der angeordnet ist, auf der ersten Schiene bewegbar zu sein; wobei die zweite Unteraufhängungsscheibe (300) eine zweite Schiene umfasst, die sich entlang einer Erstreckungsrichtung der spezifischen zweiten Unteraufhängungsscheibe (300) selbst erstreckt, sowie einen zweiten Gleiter (320), der angeordnet ist, auf der zweiten Schiene bewegbar zu sein, wobei die zweiten Unteraufhängungsscheiben (300) an dem ersten Gleiter (120) befestigt sind, wobei der zweite Gleiter (320) zur Befestigung eines Roboterarms (11) daran ausgebildet ist.

9. Aufhängungsscheiben-Positionierungsmechanismus (10) nach Anspruch 1, wobei, wenn die primären Aufhängungsscheiben (100) um die primäre Drehwelle (A1) rotieren, der Winkel zwischen zwei beliebigen primären Aufhängungsscheiben (100) nicht weniger als 60° ist.

10. Aufhängungsscheiben-Positionierungsmechanismus (10) nach Anspruch 1, wobei der Begrenzungsmechanismus ausgebildet ist, wenn der Winkel zwischen den primären Aufhängungsscheiben (100) infolge der Drehung der ersten der primären Aufhängungsscheiben (100) relativ zur zweiten der primären Aufhängungsscheiben (100) 60° erreicht, die zweite primäre Aufhängungsscheibe (100) anzutreiben, der ersten primären Aufhängungsscheibe (100) zu folgen, sich zu drehen.

11. Aufhängungsscheiben-Positionierungsmechanismus (10) nach Anspruch 1, umfassend drei primäre Aufhängungsscheiben (100), die ausgebildet sind, unabhängig voneinander um die primäre Drehwelle (A1) zu rotieren, wobei an jeder der primären Aufhängungsscheiben (100) eine erste Unteraufhängungsscheibe (200) befestigt ist, wobei jede erste Unteraufhängungsscheibe zur Befestigung mindestens eines Roboterarms (11) daran ausgebildet ist.

12. Aufhängungsscheiben-Positionierungsmechanismus (10) nach Anspruch 1, wobei mindestens eine der primären Aufhängungsscheiben (100) mit einem Kupplungsmechanismus ausgestattet ist, der es ermöglicht, die primäre Drehwelle (A1) jeder primären Aufhängungsscheibe (100) mit einer anderen Primäraufhängungsscheibe (100) zu verbinden oder sich von dieser zu lösen.

13. Aufhängungsscheiben-Positionierungsmechanismus (10) nach Anspruch 2, umfassend mindestens zwei erste Unteraufhängungsscheiben (200), wobei die erste(n) Unteraufhängungsscheibe(n) (200) mit einem Kupplungsmechanismus versehen ist (sind), der es der Unterdrehwelle (A2) jeder ersten Unteraufhängungsscheibe (200) ermöglicht, sich mit einer anderen Primäraufhängungsscheibe (100) zu verbinden oder sich von dieser zu lösen.

14. Aufhängungsscheiben-Positionierungsmechanismus (10) nach Anspruch 1, wobei die mindestens zwei primären Aufhängungsscheiben (100) ausgebildet sind, synchron in der gleichen Richtung um die primäre Drehwelle (A1) drehbar zu sein.

15. Chirurgischer Roboter (1), umfassend den Aufhängungsscheiben-Positionierungsmechanismus (10) gemäß einem der Ansprüche 1 bis 14, eine Vielzahl von Roboterarmen (11) und einen Aufhängungsarm (12),
wobei eine der primären Aufhängungsscheiben (100) mit seitlichen Armen in dem Aufhängungsscheiben-Positionierungsmechanismus (10) drehbar um die primäre Drehwelle (A1) an dem Aufhängungsarm (12) befestigt ist, wobei jede der anderen primären Aufhängungsscheiben (100) mit seitlichen Armen in dem Aufhängungsscheiben-Positionierungsmechanismus (10) drehbar an der primären Aufhängungsscheibe (100) befestigt ist, die um die primäre Drehwelle (A1) an dem Aufhängungsarm (12) befestigt ist, und wobei Enden der Arme der primären Aufhängungsscheiben (100) mit mindestens einem Roboterarm (11) daran befestigt sind, und wobei jeder der Roboterarme (11) drehbar an der entsprechenden primären Aufhängungsscheibe (100) befestigt ist.

## Revendications

1. Mécanisme de positionnement de disques de suspension (10), comprenant au moins deux disques de suspension primaires (100) dotés de bras latéraux, deux des disques de suspension primaires (100) étant fixés de manière rotative à une extrémité de suspension autour d'un seul arbre rotatif primaire (A1), les extrémités des bras des disques de suspension primaires (100) étant configurées pour la fixation d'au moins un bras de robot (11),
deux quelconques des disques de suspension primaires (100) sont pourvus entre eux d'un mécanisme de limitation, le mécanisme de limitation étant configuré pour, lorsqu'un angle entre les disques de suspension primaires (100) atteint une certaine valeur suite à la rotation d'un premier des disques de suspension primaires (100) par rapport à un second des disques de suspension primaires (100), entraîner le second disque de suspension primaire (100) à suivre le premier disque de suspension primaire (100) pour tourner.

2. Mécanisme de positionnement de disque de suspension (10) selon la revendication 1, comprenant en outre au moins un premier disque de sous-suspension (200), le ou les premiers disques de sous-suspension (200) étant fixés de manière rotative au ou aux disques de suspension primaires respectifs (100) autour d'un arbre de sous-rotation respectif (A2), l'arbre de sous-rotation étant parallèle à l'arbre de rotation primaire (A1), au moins l'un des disques de suspension primaires (100) étant fixé au ou aux premiers disques de sous-suspension (200), chacun des au moins premiers disques de sous-suspension (200) étant respectivement configuré pour la fixation du ou des bras de robot (11) à celui-ci.

3. Mécanisme de positionnement de disque de suspension (10) selon la revendication 2, dans lequel chacun desdits disques de suspension primaires (100) est fixé à celui-ci avec l'un des premiers disques de sous-suspension (200), et chaque premier disque de sous-suspension (200) est configuré pour une fixation rotative à celui-ci d'un bras de robot (11) autour de l'arbre sous-rotatif (A2).

4. Mécanisme de positionnement de disque de suspension (10) selon la revendication 1, dans lequel chacun des disques de suspension primaires (100) s'étend dans une direction perpendiculaire à l'arbre rotatif primaire (A1), le mécanisme de positionnement de disque de suspension (10) comprenant en outre au moins un second sous-disque de suspension (300) chacun fixé de manière mobile à l'un des disques de suspension primaires (100) le long d'une direction d'extension du disque de suspension primaire (100), chacun desdits seconds sous-disques de suspension (300) étant configuré pour la fixation d'au moins un bras de robot (11) à celui-ci.

5. Mécanisme de positionnement de disque de suspension (10) selon la revendication 4, dans lequel chacun des disques de suspension primaires (100) est fixé à celui-ci avec au moins deux seconds disques de sous-suspension (300), les seconds disques de sous-suspension (300) fixés au même disque de suspension primaire (100) sont espacés les uns des autres le long de la direction d'extension du disque de suspension primaire spécifique (100).

6. Mécanisme de positionnement de disque de suspension (10) selon la revendication 5, comprenant deux disques de suspension primaires (100) et quatre seconds disques de sous-suspension (300), chacun des disques de suspension primaires (100) étant fixé à celui-ci avec deux des seconds disques de sous-suspension (300).

7. Mécanisme de positionnement de disque de suspension (10) selon la revendication 4, dans lequel lesdits seconds disques de sous-suspension (300) s'étendent dans une direction perpendiculaire à l'arbre rotatif primaire (A1), une direction d'extension du second disque de sous-suspension (300) étant disposée à un angle avec la direction d'extension du disque de suspension primaire spécifique (100), et chaque second disque de sous-suspension (300) étant configuré pour la fixation mobile à celui-ci d'au moins un bras de robot (11) le long de la direction d'extension du second disque de sous-suspension spécifique (300).

8. Mécanisme de positionnement de disque de suspension (10) selon la revendication 7, dans lequel lesdits disques de suspension primaires (100) comprennent un premier rail s'étendant le long d'une direction d'extension du disque de suspension primaire spécifique (100) lui-même et un premier coulisseau (120) disposé de manière à être mobile sur le premier rail ; dans lequel ledit second sous-disque de suspension (300) comprend un second rail s'étendant le long d'une direction d'extension du second sous-disque de suspension spécifique (300) lui-même et un second coulisseau (320) disposé de manière à être mobile sur le second rail, lesdits seconds sous-disques de suspension (300) étant fixés au premier coulisseau (120), le second coulisseau (320) étant configuré pour la fixation d'un bras de robot (11) à celui-ci.

9. Mécanisme de positionnement de disque de suspension (10) selon la revendication 1, dans lequel lorsque les disques de suspension primaires (100) tournent autour de l'arbre rotatif primaire (A1), l' angle entre deux quelconques des disques de suspension primaires (100) n'est pas inférieur à 60°.

10. Mécanisme de positionnement de disque de suspension (10) selon la revendication 1, dans lequel le mécanisme de limitation est configuré pour, lorsque l'angle entre les disques de suspension primaires (100) atteint 60° en raison de la rotation du premier des disques de suspension primaires (100) par rapport au second des disques de suspension primaires (100), entraîner le second disque de suspension primaire (100) à suivre le premier disque de suspension primaire (100) en rotation.

11. Mécanisme de positionnement de disque de suspension (10) selon la revendication 1, comprenant trois disques de suspension primaires (100) configurés pour pouvoir tourner indépendamment autour de l'arbre rotatif primaire (A1), chacun des disques de suspension primaires (100) étant fixé à celui-ci avec un premier disque de sous-suspension (200), chaque premier disque de sous-suspension étant configuré pour être fixé à celui-ci avec au moins un bras de robot (11).

12. Mécanisme de positionnement de disque de suspension (10) selon la revendication 1, dans lequel au moins un des disques de suspension primaires (100) est pourvu d'un mécanisme d'embrayage permettant à l' arbre de rotation primaire (A1) de chaque disque de suspension primaire de tourner (100) à relier ou à libérer d'un autre disque de suspension primaire (100).

13. Mécanisme de positionnement de disque de suspension (10) selon la revendication 2, comprenant au moins deux premiers disques de sous-suspension (200), dans lequel le(s) premier(s) disque(s) de sous-suspension (200) est(sont) pourvu(s) d'un mécanisme d'embrayage, qui permet à l' arbre sous-rotatif (A2) de chaque premier disque de sous-suspension (200) à relier ou à libérer d'un autre premier disque de sous-suspension (200).

14. Mécanisme de positionnement de disque de suspension (10) selon la revendication 1, dans lequel les au moins deux disques de suspension primaires (100) sont configurés pour pouvoir tourner de manière synchrone autour de l'arbre rotatif primaire (A1) dans la même direction.

15. Robot chirurgical (1), comprenant le mécanisme de positionnement de disque de suspension (10) selon l'une quelconque des revendications 1 à 14, une pluralité de bras de robot (11) et un bras de suspension (12),
dans lequel l'un des disques de suspension primaires (100) avec des bras latéraux dans le mécanisme de positionnement de disque de suspension (10) est fixé de manière rotative au bras de suspension (12) autour de l'arbre rotatif primaire (A1), dans lequel chacun des autres disques de suspension primaires (100) avec des bras latéraux dans le mécanisme de positionnement de disque de suspension (10) est fixé de manière rotative au disque de suspension primaire (100) fixé au bras de suspension (12) autour de l'arbre rotatif primaire (A1), et dans lequel les extrémités des bras des disques de suspension primaires (100) sont fixées à ceux-ci avec au moins un bras de robot (11), et chacun desdits bras de robot (11) est fixé de manière rotative au disque de suspension primaire correspondant (100).
